(19) Europäisches Patentamt
European Patent Office
Office européen des brevets

(11) Publication number: **0 580 409 A2**

(12) # EUROPEAN PATENT APPLICATION

(21) Application number: **93305702.8**

(51) Int. Cl.$^5$: **A61K 7/06**, A61K 7/48

(22) Date of filing: **20.07.93**

(30) Priority: **20.07.92 GB 9215354**

(43) Date of publication of application:
**26.01.94 Bulletin 94/04**

(84) Designated Contracting States:
**AT BE CH DE DK ES FR GB GR IE IT LI NL PT SE**

(71) Applicant: **UNILEVER PLC**
**Unilever House Blackfriars**
**London EC4P 4BQ (GB)**
(84) **GB IE**

(71) Applicant: **UNILEVER N.V.**
**Weena 455**
**NL-3013 AL Rotterdam (NL)**
(84) **BE CH DE DK ES FR GR IT LI NL PT SE AT**

(72) Inventor: **Gibson, Walter Thomas**
**8 Braid Court**
**Wellingborough, Northampton NN8 6PF (GB)**
Inventor: **Jones, Keith**
**"Bankdale", Dibbinsdale Road**
**Bromborough, Wirral, Merseyside L63 0HF (GB)**
Inventor: **Gough, Anthony David**
**44 Redcar Drive**
**Eastham, Wirral, Merseyside L62 8HE (GB)**
Inventor: **Nation, Jayne Elizabeth**
**Flat 3, 32 Carlton Road**
**Oxton, Birkenhead L42 9NQ (GB)**
Inventor: **Jenkins, Gail**
**77 Dingle road**
**Rushden, Northampton NN10 9UE (GB)**
Inventor: **Taylor, Anthony Philip**
**Lindendreef 10**
**NL-3137 CM Vlaardingen (NL)**

(74) Representative: **Ford, Michael Frederick et al**
**MEWBURN ELLIS 2 Cursitor Street**
**London EC4A 1BQ (GB)**

(54) **Cosmetic composition containing DOPA derivatives.**

(57) A composition for topical application to human hair or skin contains a chemical analogue of dihydroxyphenyl alanine (DOPA). This chemical analogue can be absorbed by skin or by a hair follicle and metabolised in-vivo, thus leading to the formation of melanin in skin or to the growth of melanin-pigmented hair. Consequently the composition can give controlled skin darkening to mimic sun-induced tanning or can bring about the growth of dar hair in place of the grey or white hair.

EP 0 580 409 A2

## FIELD OF INVENTION

The invention relates to a composition suitable for topical application to human skin, particularly the scalp, which can enhance the darkening of hair on regrowth. The composition is also suitable for topical application to human skin, especially normally exposed areas, where controlled skin darkening to mimic sun-induced tanning is desired.

## BACKGROUND TO THE INVENTION AND PRIOR ART

The darkening of human hair is widely seen as desirable, particularly when hair becomes grey or white with advancing age, or even prematurely in the young adult. A variety of remedies is available to reverse the undesirable lightening of hair and these rely on topical treatment of the hair with colourants or dyes, which can provide temporary or permanent darkening of the hair to a desired shade.

Colourants or dyes generally act by coating or penetrating the shaft of the hair external to the skin. The darkening effect can then last until growth of new hair reveals the original colour of the hair and retreatment with a colourant or dye is then necessary to maintain the desired darkening.

The external application of colourants and dyes can be inconvenient, in as much as dye can transfer to the hands during application, or the scalp can take up some of the dye to an undesirable degree.

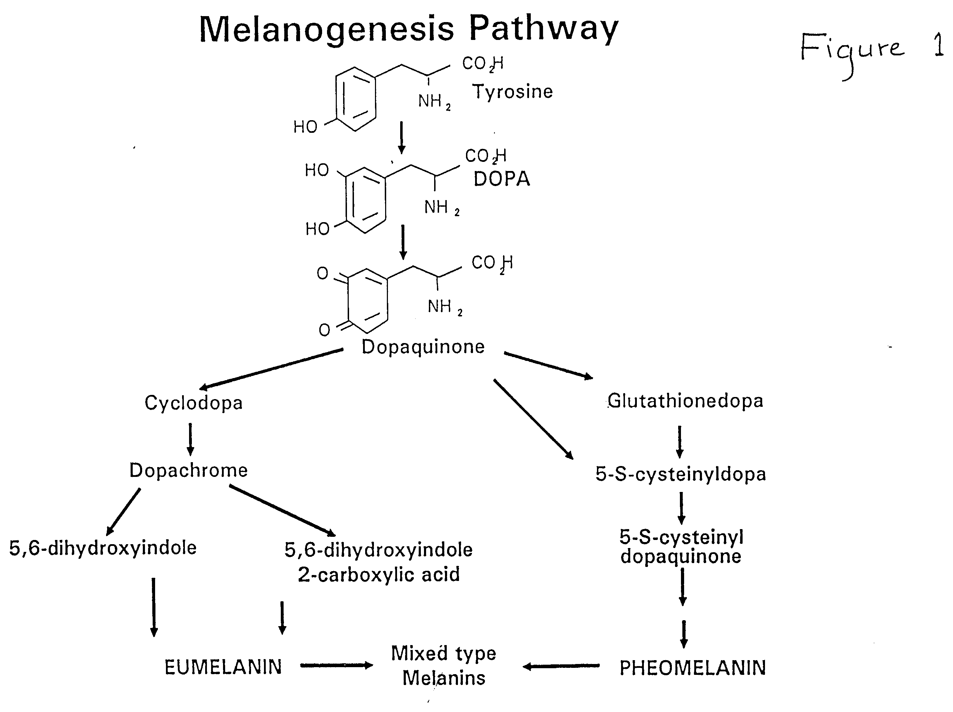

The natural dark colour of hair is due to the presence of a blend of melanins, principally eumelanin and pheomelanin, hereinafter referred to collectively as "melanin", derived initially from tyrosine via the melanogenesis pathway. In accordance with this pathway, tyrosine is converted first to dihydroxyphenylalanine, hereinafter referred to as "DOPA", and then to DOPAquinone under the influence of tyrosinase. DOPAquinone is then converted via a variety of further intermediates either to eumelanin or pheomelanin in accordance with the pathway diagram shown in Figure 1.

Now, in order to promote more effective darkening of hair on regrowth, or indeed controlled darkening of the skin to mimic tanning, it would be advantageous to select an intermediate in the melanogenesis pathway which is closer to melanin than tyrosine. Many of these intermediates are however unstable, costly to synthesise or lack the necessary ability to penetrate through the skin, especially the skin of the scalp, to reach the immediate environment of the hair bulb, where their conversion to melanin in the growing hair can be achieved.

DOPA itself is unstable and cannot be used in a product intended for topical application to the skin, as it tends to oxidise prematurely to give brown coloured derivatives which are then unsuitable for darkening hair on regrowth or for darkening the skin. We have, however, discovered that certain derivatives of DOPA which are stable to autoxidation can be employed topically and are more readily converted by the growing hair, or the skin, to melanin than is tyrosine itself.

We have now approached the problem of darkening skin and/or hair by applying topically to skin, and in particular to the scalp, a stable derivative of DOPA which we believe is then converted to melanin, to provide enhanced pigmentation in the growing hair follicle before it emerges from the skin surface. By the same mechanism, skin can be gradually darkened following topical application of this stable derivative of DOPA.

Topical application of these derivatives of DOPA has the advantage that they do not stain the hands during application to the desired area, especially where darker hair on regrowth is desired. Darkening of the skin, if desired, will however normally occur gradually after repeated applications.

## DEFINITION OF THE INVENTION

Accordingly, the invention provides a preserved composition suitable for topical application to mammalian skin for darkening hair on regrowth, which composition comprises:

i. an effective amount of a substituted DOPA as a melanin precursor, chosen from:

(a) mono and/or di-substituted ester and formate derivatives of DOPA having the structure (10):

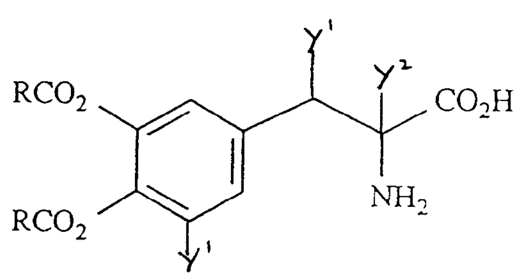

(10)

where R is -H, a branched or unbranched, alkyl or alkenyl group having from 1 to 20 carbon atoms, or an amino acid or peptide fragment,
$Y^1$ is -H or -OH, and
$Y^2$ is -H or $-CH_3$;
(b) mono and/or di-substituted carbonate derivatives of DOPA having the structure (11):

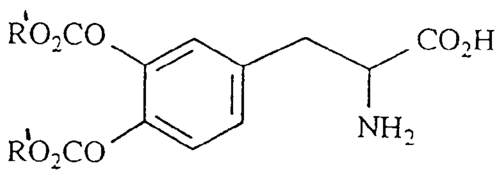

(11)

where $R^1$ is a branched or unbranched, alkyl or alkenyl group having from 1 to 20 carbon atoms;
(c) mono and/or di-substituted urethane derivatives of DOPA having the structure (12):

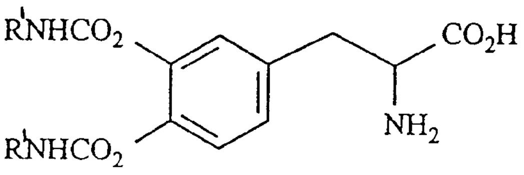

(12)

(d) mono and/or di-substituted ether derivatives of DOPA having the structure (13):

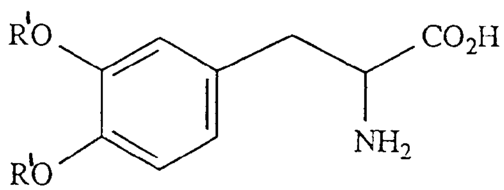

(13)

(e) mono and/or di-substituted phosphate and/or sulphate derivatives of DOPA having the structure (14):

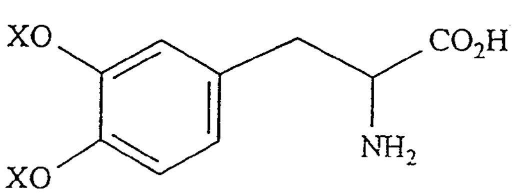

(14)

where one X is $-PO_3H_2$ or $-SO_3H$ and the other X is -H, $-PO_3H_2$ or $-SO_3H$;
(f) acetal and ketal derivatives of DOPA having the structure (15):

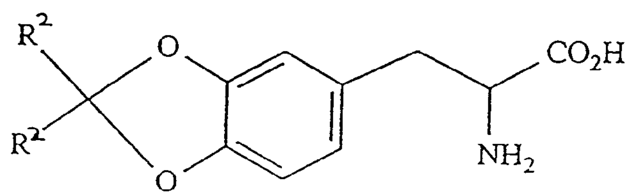

(15)

where $R^2$ is chosen from -H, alkyl and phenyl;
(g) cyclic carbonate derivatives of DOPA having the structure (16):

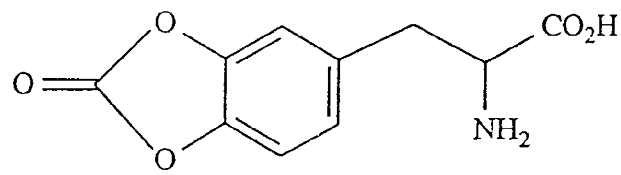

(16)

(h) amino substituted derivatives of DOPA having the structure (17):

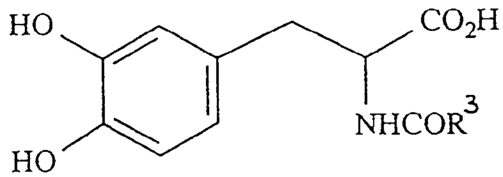

(17)

where $R^3 = R^1$ or an amino acid residue;

(i) carboxylate substituted derivatives of DOPA having the structure (18):

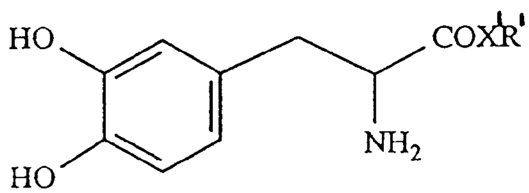

(18)

where $X^1$ is an NH amide (especially an amino acid or peptide) linkage;

(j) linked amino and carboxylate substituted derivatives of DOPA having the structure (19):

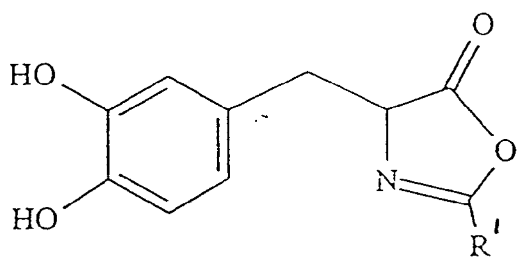

(19)

(k) structural analogues of DOPA having the structure (20):

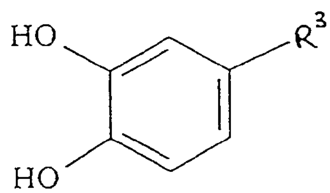

(20)

where $R^3$ is chosen from:

(i)

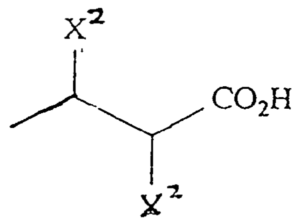

where $X^2$ is the same or different and is chosen from -H, -OH, $-NH_2$ or -SH

(ii)

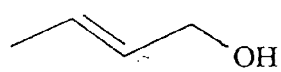

(iii)

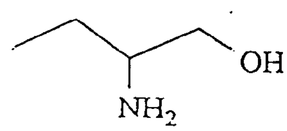

and

(iv)

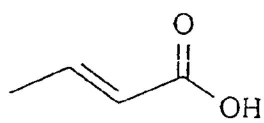

(l) structural analogues of DOPA having the structure (21):

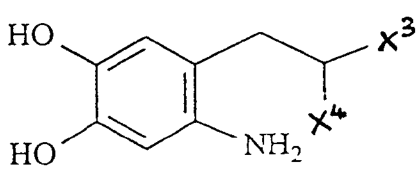

(21)

where

$X^3$ is -OH or $OR^1$

$X^4$ is =O or $OR^1$

(m) C-homologues of DOPA having the structure (22):

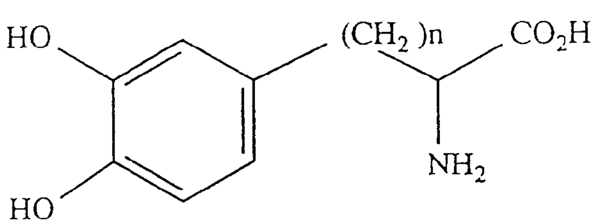

(22)

where n is an integer of from 1 to 3;

(n) short chain and hetero atom analogues of DOPA having the structure (23):

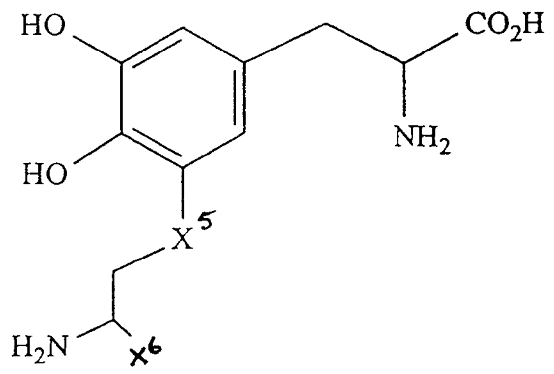

(23)

where

$X^5$ is S, O or NH

$X^6$ is H or COOH; and

(n) a glutathione derivative having the structure (24):

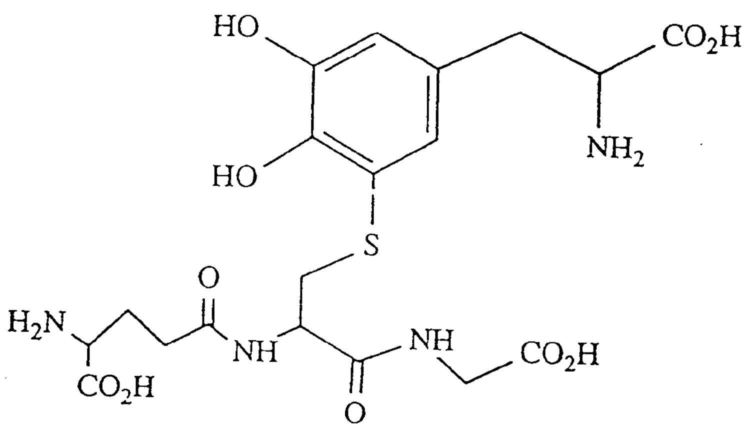

(24)

ii) a cosmetically acceptable vehicle for the substituted DOPA.

DISCLOSURE OF THE INVENTION

By "preserved composition", is meant that the composition is free from microbial contaminants capable of resulting in microbial spoilage of the composition and/or biodegradation of the substituted DOPA.

The substituted DOPA

The substituted DOPA is chosen from those groups of DOPA derivatives having the structures (10) to (24) as herein defined:

Some preferred examples of the substituted DOPAs are:

3,4-dihydroxyphenylserine, having the structure (50):

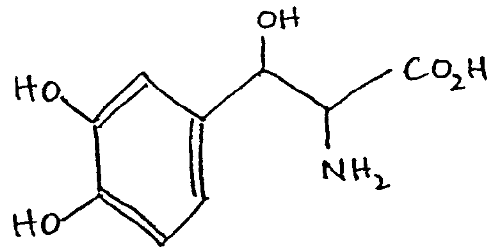

(50)

2,4,5,-trihydroxyphenylalanine, having the structure (51):

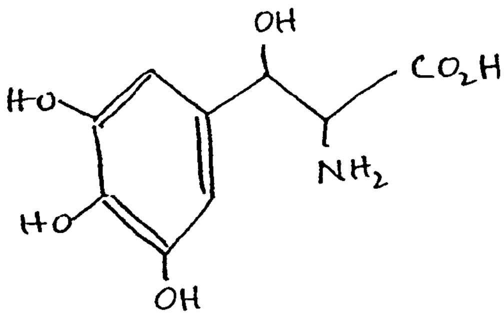

(51)

2-methyl-3-(3,4-dihydroxyphenylalanine), having the structure (52):

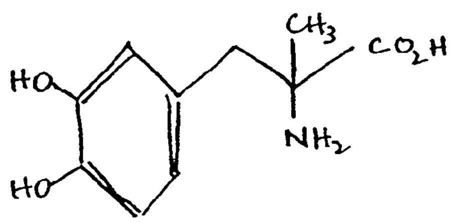

(52)

Another possibility is methoxytyrosine having the structure (53):

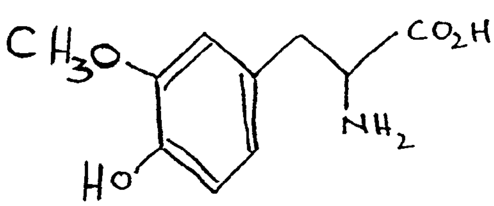

(53)

and its isomer 3-hydroxy-4-methoxyphenyl alanine of structure:

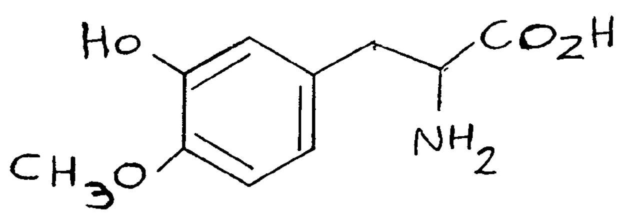

Mixtures comprising two or more of the substituted DOPAs can also be employed in the composition according to the invention.

The total amount of substituted DOPA present in the composition according to the invention is sufficient to give a detectable increase in skin darkening or in hair darkening on regrowth, following topical application to the skin, especially the scalp, compared with the untreated skin.

The sufficient amount will depend on the effectiveness of the substituted DOPA, some being more effective than others, but in general, an amount of from 0.0001 to 99.9%, preferably from 1 to 50% by weight of the composition will provide an adequate dose to the skin or to the hair bulb and hair follicle after topical application to the scalp.

Preservation of the composition

The composition according to the invention is preferably preserved in such a manner that it will enjoy an extended shelf life following manufacture and prior to sale and use. Ideally the composition will have an indefinite shelf life.

It is accordingly apparent that the substituted DOPA is likely to be prone to attack by bacteria, moulds and fungi and other microbial influences, particularly at pH values near neutrality that characterise the preferred composition. The shelf-life of the composition can therefore be unacceptably short due to the biodegradation of the activator unless steps are taken to preserve the composition.

In order to be preserved, the composition should preferably be free, or substantially free, from viable microbial contaminants that are capable of resulting in microbial spoilage of the composition, and/or biodegradation of the activator prior to topical application of the composition to mammalian skin or hair. It is to be understood, however, that the invention is also concerned with compositions, as herein defined, which may contain viable but dormant microorganisms, such as bacterial spores, provided that the condition of preservation do not result in substantial proliferation of the microorganisms prior to use of the composition.

Examples of methods that can be employed to achieve preservation of the composition, includes the following:

(i) Sterilisation

The composition according to the invention can be preserved by sterilisation to remove or kill substantially all viable microbial contaminants. This can be achieved for example by irradiation using a lethal dose of gamma rays, by heat sterilisation or by ultrafiltration using techniques that are well established in the pharmaceutical industry.

(ii) Chemical Preservative

The composition according to the invention can also be preserved by including in it a chemical preservative which functions to prevent the growth of or kill bacteria, fungi or other microorganisms.

Examples of chemical preservatives include ethanol, benzoic acid, sodium benzoate, sorbic acid, potassium sorbate, sodium propionate and the methyl, ethyl, propyl and butyl esters of p-hydroxybenzoic acid. The amount of chemical preservative that can be incorporated in the composition according to the invention will generally be from 0.05 to 5%, preferably from 0.1 to 2% by weight, the amount chosen being sufficient to arrest microbial proliferation.

(iii) Water activity depressants

The composition according to the invention can also be preserved by the inclusion of a water activity depressant such as glycerol, propylene glycol, sorbitol, sugars and salts, for example alkali metal halides, sulphates and carboxylates. When employing a water activity depressant, sufficient should be incorporated in the composition according to the invention to reduce the water activity ($\alpha_W$) from 1 to $< 0.9$, preferably to $< 0.85$ and most preferably $< 0.8$, the lowest of these values being that at which yeasts, moulds and fungi will not proliferate.

The Vehicle

The composition according to the invention also comprises a solid, semi-solid or liquid cosmetically and/or physiologically acceptable vehicle, to enable the substituted DOPA to be conveyed to the skin or hair at an appropriate dilution. The nature of the vehicle will depend upon the method chosen for administration of the composition to the skin. The vehicle can itself be inert or it can possess physiological or pharmaceutical benefits of its own.

It should be explained that vehicles are substances which can act as dilutants, dispersants, or solvents for the substituted DOPA, which therefore ensure that it can be applied to and distributed evenly over the hair and/or skin, especially the scalp, at an appropriate concentration. The vehicle is preferably one which can aid penetration of the substituted DOPA into the skin, particularly to reach the immediate environment of the hair bulb, thereby improving its ability to stimulate the production of melanin. The rôle and identity of selected vehicles as activity enhancers is described later in this specification.

Compositions according to this invention can include water as a vehicle, usually with at least one other cosmetically-acceptable vehicle.

Vehicles other than water that can be used in compositions according to the invention can include liquids or solids as emollients, solvents, humectants, thickeners and powders. Examples of each of these types of vehicles, which can be used singly or as mixtures of one or more vehicles, are as follows:

Emollients, such as stearyl alcohol, glyceryl monoricinoleate, glyceryl monostearate, propane-1,2-diol, butane-1,3-diol, mink oil, cetyl alcohol, ispropyl isostearate, stearic acid, isobutyl palmitate, isocetyl stearate, oleyl alcohol, isopropyl laurate, hexyl laurate, decyl oleate, octadecan-2-ol, isocetyl alcohol, eicosanyl alcohol, behenyl alcohol, cetyl palmitate, dimethylpolysiloxane, di-n-butyl sebacate, isopropyl myristate, isopropyl palmitate, isopropyl stearate, butyl stearate, polyethylene glycol, triethylene glycol, lanolin, cocoa butter, corn oil, cotton seed oil, tallow, lard, olive oil, palm kernel oil, rapeseed oil, safflower seed oil, soybean oil, sunflower seed oil, olive oil, sesame seed oil, coconut oil, arachis oil, castor oil, acetylated lanolin alcohols, petroleum, mineral oil, butyl myristate, isostearic acid, palmitic acid, isopropyl linoleate, lauryl lactate, myristyl lactate, decyl oleate, myristyl myristate;

Propellants, such as propane, butane, isobutane, dimethyl ether, carbon dioxide, nitrous oxide;

Solvents, such as ethyl alcohol, methylene chloride, isopropanol, acetone, castor oil, ethylene glycol monoethyl ether, diethylene glycol monobutyl ether, diethylene glycol monoethyl ether, dimethyl sulphoxide, dimethyl formamide, tetrahydrofuran;

Humectants, such as glycerin, sorbitol, sodium 2-pyrrolidone-5-carboxylate, soluble collagen, dibutyl phthalate, gelatin;

Powders, such as chalk, talc, fullers earth, kaolin, starch, gums, colloidal silicon dioxide, sodium polyacrylate, tetra alkyl and/or trialkyl aryl ammonium smectites, chemically modified magnesium aluminium silicate, organically modified montmorillonite clay, hydrated aluminium silicate, fumed silica, carboxyvinyl polymer, sodium carboxymethyl cellulose, ethylene glycol monostearate.

The total amount of the vehicle present in the composition according to the invention is from 0.1 to 99.999%, preferably from 50 to 99% by weight of the composition. The vehicle can accordingly form the balance of the composition.

## OPTIONAL SKIN BENEFIT MATERIALS AND COSMETIC ADJUNCTS

A particularly convenient form of the composition according to the invention is a shampoo with or without hair conditioning properties.

### Surfactant

When the composition is to be provided in the form of a shampoo, it will normally comprise a surfactant chosen from anionic, nonionic or amphoteric surfactant or mixtures thereof.

Suitable anionic surfactants are the alkyl sulphates, alkyl ether sulphates, alkaryl sulphonates, alkyl succinates, alkyl sulphosuccinates, N-alkoyl sarcosinates, and alpha-olefin sulphonates, especially their sodium, magnesium, ammonium and mono-, di- and triethanolamine salts. The alkyl groups generally contain from 8 to 18 carbon atoms and may be unsaturated. The alkyl ether sulphates may contain from one to 10 ethylene oxide or propylene oxide units per molecule, and preferably contain 2 to 3 ethylene oxide units per molecule.

Examples of suitable anionic surfactants include sodium lauryl sulphate, sodium oleyl succinate, ammonium lauryl sulphosuccinate, ammonium lauryl sulphate, sodium dodecylbenzene sulphonate, triethanolamine dodecylbenzene sulphonate and sodium N-lauryl sarcosinate. The most preferred anionic surfactants are sodium lauryl sulphate, sodium lauryl ether sulphate 2EO and 3EO, ammonium lauryl sulphate and ammonium lauryl ether sulphate 1EO, 2EO and 3EO.

The nonionic surfactants suitable for use in the composition of the invention may include condensation products of aliphatic ($C_8$-$C_{18}$) primary or secondary linear or branched chain alcohols or phenols with alkylene oxides, usually ethylene oxide and generally 6-30 EO.

Other suitable nonionics include mono or di alkyl alkanolamides or alkyl polyglucosides. Examples include

coco mono or diethanolamide, coco mono isopropanolamide, and coco di glucoside.

The amphoteric surfactants suitable for use in the composition of the invention may include alkyl amine oxides, alkyl betaines, alkyl amidopropyl betaines, alkyl mono- or dialkanolamides, alkyl sulphobetaines, alkyl glycinates and alkyl carboxyglycinates, wherein the alkyl groups have from 8 to 18 carbon atoms. Examples include lauryl amine oxide, cocomonoethanolamide, cocodiethanolamide, cocamidopropyl betaine, cocodimethyl sulphopropyl betaine and preferably cocobetaine.

The surfactants are present in the shampoo composition of the invention in an amount of from 2 to 40% by weight, and preferably from 5 to 30% by weight.

If an amount of less than 2% by weight of surfactant is present in the composition, inadequate foaming is achieved, and if more than 40% by weight is present, no further increase in cleansing power or foaming ability is observed.

## Conditioning Agent

When the composition is to be provided as a hair conditioner, a "leave-on product" which is generally not rinsed from the hair after application, it will normally comprise a cationic conditioning agent. Alternatively, the composition of the invention can be a shampoo-conditioner which is usually rinsed from the hair after use.

Suitable cationic conditioning agents, whether intended for "leave-on" or "rinse-off" products, include the cationic cellulose ethers described in US Patent Nos. 3 816 616 and 4 272 515 and which are available commercially from Union Carbide Corporation as Polymer JR. Other suitable materials are the cationic polygalactomannan gum derivatives describes in US Patent No. 4 298 494 which are commercially available under the trade mark Jaguar from Celanese-Stein Hall. An example of a suitable material has the CTFA designation guar hydroxypropyltrimonium chloride and is available under the name Jaguar C13S, which has a degree of substitution of the cationic groups of about 0.13. Other suitable materials include that known as Jaguar C17 (degree of substitution of about 0.25 to 0.31), and Jaguar C16 which is hydroxypropylated cationic guar derivative containing hydroxypropyl substituent groups as well as cationic quaternary ammonium groups. In Jaguar C16, the degree of substitution is 0.11 to 0.16 and the moles of substitution of hydroxypropyl groups is 0.8 to 1.1.

Other cationic conditioning agents useful in the compositions of the present invention include cationic polyamide polymers such as the low molecular weight adipic acid/diethylene-triamine polyamide and the copolymers of vinylpyrrolidone and dimethylaminoethyl methacrylate quaternised with dimethyl sulphate (Gafquat 755, GAF Corporation) described in US Patent No. 4 080 310; the graft cationic copolymer containing N-vinylpyrrolidone, dimethylamonoethyl methacrylate and polyethylene glycol described in US Patent No. 4 048 301; the mineral acid salts of the amono-alkyl esters of homo- and copolymers of unsaturated carboxylic acids having from 3 to 5 carbon atoms described in US Patent No. 4 009 256; and the polymers of etherified starch described in US Patent No. 3 186 911.

The high molecular weight polymers sold under the trade mark Merquat by Merck & Co. Inc., are cationic polymers which are also suitable for use in the compositions of the invention. Representative ones are Merquat 100, a highly charged cationic dimethyldiallylammonium chloride homopolymer, and Merquat 550, a highly charged cationic copolymer prepared with dimethyldiallylammonium chloride and acrylamide. These materials are designated in the CFTA dictionary as Quaternium-40 and Quaternium-41, respectively.

Cationic surfactants such as mono-, di- and tri-alkyl quaternary ammonium salts may also be used as the cationic conditioning agent in the compositions of the invention. Suitable examples are cetyl trimethylammonium chloride, cetyl trimethylammonium bromide and stearyltrimethylammonium chloride.

The cationic conditioning agent when employed is preferably present in the composition of the invention in an amount of from 0.01 to 5% by weight, most preferably in an amount of from 0.2 to 3% by weight.

## Emulsion

A further convenient form of the composition according to the invention is an emulsion, in which case an oil or oily material will normally be present, together with an emulsifier to provide either a water-in-oil emulsion or an oil-in-water emulsion, depending largely on the average hydrophilic-lyophilic balance (HLB) of the emulsifier employed.

## Oil or oily material

The composition according to the invention can optionally comprise one or more oils or other materials having the properties of an oil.

Examples of suitable oils include mineral oil and vegetable oils, and oil materials, such as those already

proposed herein as emollients. Other oils or oily materials include silicone oils, both volatile and non-volatile, such as polydimethyl siloxanes.

The oil or oily material, when present for the purposes for forming an emulsion, will normally form up to 90%, preferably from 10 to 80% by volume of the composition.

## Emulsifier

The composition according to the invention can also optionally comprise one or more emulsifiers the choice of which will normally determine whether a water-in-oil or and oil-in-water emulsion is formed.

When a water-in-oil emulsion is required, the chosen emulsifier or emulsifiers should normally have an average HLB value of from 1 to 6. When an oil-in-water emulsion is required, a chosen emulsifier or emulsifiers should have an average HLB value of >6.

Examples of suitable emulsifiers are set below in Table 1 in which the chemical name of the emulsifiers is given together with an example of a trade name as commercially available, and the average HLB value.

Table 1

| Chemical Name of Emulsifier | Trade Name | HLB Value |
|---|---|---|
| Sorbitan trioleate | Arlacel 85 | 1.8 |
| Sorbitan tristearate | Span 65 | 2.1 |
| Glycerol monooleate | Aldo MD | 2.7 |
| Glycerol monostearate | Atmul 84S | 2.8 |
| Glycerol monolaurate | Aldo MC | 3.3 |
| Sorbitan sesquioleate | Arlacel 83 | 3.7 |
| Sorbitan monooleate | Arlacel 80 | 4.3 |
| Sorbitan monostearate | Arlacel 60 | 4.7 |
| Poloxyethylene (2) stearyl ether | Brij 72 | 4.9 |
| Poloxyethylene sorbitol beeswax derivative | G-1702 | 5 |
| PEG 200 dilaurate | Emerest 2622 | 6.3 |
| Sorbitan monopalmitate | Arlacel 40 | 6.7 |
| Polyoxyethylene (3.5) nonyl phenol | Emulgen 903 | 7.8 |
| PEG 200 monostearate | Tegester PEG 200 MS | 8.5 |
| Sorbitan monolaurate | Arlacel 200 | 8.6 |
| PEG 400 dioleate | Tegester PEG 400-DO | 8.8 |
| Polyoxyethylene (5) monostearate | Ethofat 60-16 | 9.0 |
| Polyoxyethylene (4) sorbitan monostearate | Tween 61 | 9.6 |
| Polyoxyethylene (4) lauryl ether | Brij 30 | 9.7 |
| Polyoxyethylene (5) sorbitan monooleate | Tween 81 | 10.0 |
| PEG 300 monooleate | Neutronyx 834 | 10.4 |

| | | |
|---|---|---|
| Polyoxyethylene (20) sorbitan tristearate | Tween 65 | 10.5 |
| Polyoxyethylene (20) sorbitan trioleate | Tween 85 | 11.0 |
| Polyoxyethylene (8) monostearate | Myrj 45 | 11.1 |
| PEG 400 monooleate | Emerest 2646 | 11.7 |
| PEG 400 monostearate | Tegester PEG 400 | 11.9 |
| Polyoxyethylene 10 monooleate | Ethofat 0/20 | 12.2 |
| Polyoxyethylene (10) stearyl ether | Brij 76 | 12.4 |
| Polyoxyethylene (10) cetyl ether | Brij 56 | 12.9 |
| Polyoxyethylene (9.3) octyl phenol | Triton X-100 | 13.0 |
| Polyoxyethylene (4) sorbitan monolaurate | Tween 21 | 13.3 |
| PEG 600 monooleate | Emerest 2660 | 13.7 |
| PEG 1000 dilaurate | Kessco | 13.9 |
| Polyoxyethylene sorbitol lanolin derivative | G-1441 | 14.0 |
| Polyoxyethylene (12) lauryl ether | Ethosperse LA-12 | 14.4 |
| PEG 1500 dioleate | Pegosperse 1500 | 14.6 |
| Polyoxyethylene (14) laurate | Arosurf HFL-714 | 14.8 |
| Polyoxyethylene (20) sorbitan monostearate | Tween | 14.9 |
| Polyoxyethylene 20 sorbitan monooleate | Tween 80 | 15.0 |
| Polyoxyethylene (20) stearyl ether | Brij 78 | 15.3 |
| Polyoxyethylene (20) sorbitan monopalmitate | Tween 40 | 15.6 |

| | | |
|---|---|---|
| Polyoxyethylene (20) cetyl ether | Brij 58 | 15.7 |
| Polyoxyethylene (25) oxypropylene monostearate | G-2162 | 16.0 |
| Polyoxyethylene (20) sorbitol monolaurate | Tween 20 | 16.7 |
| Polyoxyethylene (23) lauryl ether | Brij 35 | 16.9 |
| Polyoxyethylene (50) monostearate | Myrj 53 | 17.9 |
| PEG 4000 monostearate | Pegosperse 4000 MS | 18.7 |

The foregoing list of emulsifiers is not intended to be limiting and merely exemplifies selected emulsifiers which are suitable for use in accordance with the invention.

It is to be understood that two or more emulsifiers can be employed if desired.

The amount of emulsifier or mixtures thereof, that optionally can be incorporated in the composition of the invention is from 1 to 50%, preferably from 2 to 20% and most preferably from 2 to 10% by weight of the composition.

Water

The composition of the invention can also comprise water, usually up to 80%, preferably from 5 to 80% by volume.

Silicone Surfactant

The composition of the invention can also optionally comprise a high molecular weight silicone surfactant which can also act as an emulsifier, in place of or in addition to the optional emulsifier(s) already mentioned.

The silicone surfactant is a high molecular weight polymer of dimethyl polysiloxane with polyoxyethylene and/or polyoxypropylene side chains, generally having a molecular weight of from 10,000 to 50,000.

The dimethyl polysiloxane polymer is conveniently provided as a dispersion in a volatile siloxane, the dispersion comprising, for example, from 1 to 20% by volume of the polymer and from 80 to 99% by volume of the volatile siloxane. Ideally, the dispersion consists of a 10% by volume of the polymer dispersed in the volatile siloxane.

Examples of the volatile siloxanes in which the polysiloxane polymer can be dispersed include polydimethyl siloxane (pentamer and/or hexamer).

A particularly preferred silicone surfactant is cyclomethicone and dimethicone copolyol, such as DC 3225C Formulation Aid available from DOW CORNING. Another is laurylmethicone copolyol, such as DC Q2-5200, also available from Dow Corning.

The amount of silicone surfactant, when present in the composition will normally be up to 25%, preferably from 0.5 to 15% by weight of the emulsion.

Activity Enhancer

The composition according to the invention can also optionally comprise an activity enhancer.

The activity enhancer can be chosen from a wide variety of molecules which can function in different ways to enhance the benefits of the substituted DOPAs. Particular classes of activity enhancers include (a) hair growth stimulants and (b) penetration enhancers, whose presence can further improve the delivery of the substituted DOPA through the stratum corneum to its site of action, particularly in the immediate environment of the hair follicle.

Some activity enhancers can also function as vehicles for the sustituted DOPA.

(a) Hair Growth Stimulants

i. Examples of substances which themselves possess the ability to stimulate or increase hair growth and so accelerate the benefits of the substituted DOPA in darkening hair on regrowth include, for example:

Benzalkonium chloride
Benzethonium chloride
Phenol
Estradiol
Diphenhydramine hydrochloride
Chlorpheniramine maleate
Chlorophyllin derivatives
Cholesterol
Salicylic acid
Cystine
Methionine
Red pepper tincture
Benzyl nicotinate
dl-Menthol
Peppermint oil
Calcium pantothenate
Panthenol
Castor oil
Hinokitiol
Prednisolone
Resorcinol

Further substances which themselves possess the ability to increase the rate of terminal hair growth include:

ii. α-1,4 esterified disaccharides described by Choay S.A. in EP-A-O 064 012, having the structure (100):

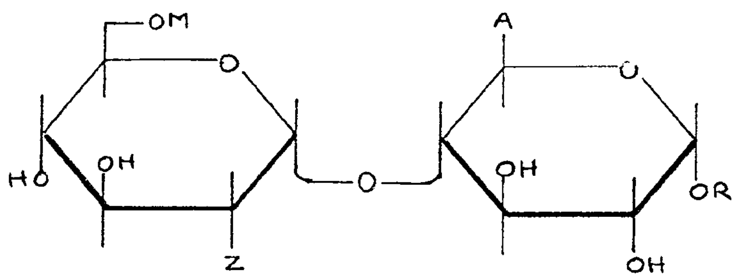

(100)

where

Z represents a functional nitrogen group, such as an azide or a group having the structure -NHB, in which B represents -H or a functional group such as acetyl or sulphate as a salt with an organic or mineral cation;

M represents -H or $SO_3M_1$, where $M_1$ is an organic or metallic cation, particularly an alkali metal; or an acetyl group;

R represents a $C_1$ to $C_4$ alkyl radical, especially methyl; or an aryl radical;

A represents a functional group such as an acid or $-COOR_1$, where R1 represents -H or a $C_1$ to $C_4$ alkyl radical, especially methyl; or a metal, especially an alkali metal;

esterified oligosaccharides as described by Unilever in EP-A-O 211 610, including at least one esterified disaccharide unit consisting of a uronic acid residue having the structure (101):

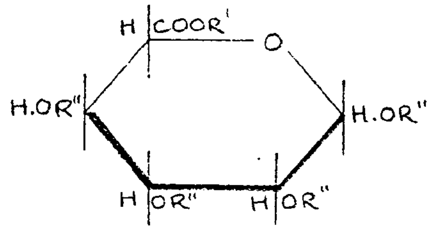

(101)

and a hexosamine residue having the structure (102):

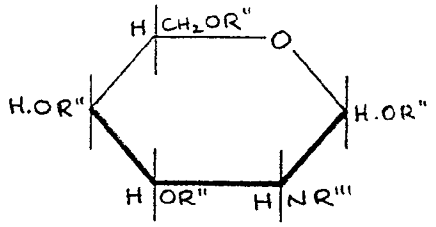

(102)

where

R' is -H, $C_3$ to $C_{10}$ alkyl or

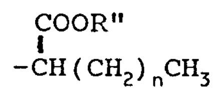

R" is -H, $C_1$ to $C_4$ alkyl, $-CO(CH_2)_mCH_3$, $-SO_3M$,
R"' is -H, $-CO(CH_2)_mCH_3$, or $-SO_3M$,
M is -H, or a metallic or organic cation
n is 0 or an integer of from 1 to 7, and
m is 0 or the integer 1 or 2;

the groups designated R" being the same or different, one R" group from each pyranose ring structure being linked by a glycosidic linkage having the configuration α-1,3, α-1,4, β-1,3 or β-1,4; and the -COOR', $-CH_2OR$" and -OR" groups being of either configuration with respect to the pyranose rings;

iii. Minoxidil glucuronides, as described by Unilever in EP-O 242 967;

iv. Minoxidil sulphates, as described by The Upjohn Co. in WO 86/04231; and

v. Minoxidil, and other derivatives thereof as described by The Upjohn Co, in US patent 4 139 619.

vi. Ethylenediaminetetraacetic acid or salts thereof, as described by Redken Laboratories, Inc. in US 4 814 351.

vii. Direct proteoglycanase inhibitors, such as 1,10-phenanthroline, as described by Unilever in EP-0 277 428.

viii. Glycosaminoglycanase inhibitors, as described by Unilever in EP-0 277 428, such as aldonolactones and esterified aldonolactones,

preferred examples of which include:

L-Galactono-1,4-lactone
L-Arabino-1,5-lactone
D-Fucono-1,5-lactone
D-Glucaro-1,4-lactone
D-Glucurono-6,3-lactone
Galactaric acid lactone
2-Acetamido-2-deoxygluconolactone
2-Acetamido-2-deoxygalactono-lactone
D-Glucaro-1,4:6,3-dilactone
L-Idaro-1,4-lactone
2,3,5-Tri-0-acetyl-D-glucaro-1,4-lactone
2,5-Di-0-acetyl-D-glucaro-1,4:6,3-dilactone.

ix. Glycosaminoglycanase inhibitors, as described by Unilever in EP 0 277 428, such as monosaccharides

and esterified monosaccharides,
preferred examples of which include:
N-Acetylglucosamine
N-Acetylgalactosamine
D-Galactosamine
D-Glucosamine-3-sulphate
N-Acetylmannosamine.

x. Glycosaminoglycan chain cellular uptake inhibitors, as described by Unilever in EP 0 277 428, such as hexuronic acid and esters thereof.

xi. Chemical inhibitors of glycosidase activity, as described by Unilever in EP 0 334 586, chosen from lactams,
preferred examples of which include:
D-glucaro-1,5-lactam,
L-Galactono-1,4-lactam,
L-Arabino-1,5-lactam,
D-Fucono-1,5-lactam,
D-Glucaro-1,4-lactam,
D-Glucurono-6,3-lactam,
1,2,5-tri-O-acetyl-D-glucurono-6,3-lactam,
2-Acetamido-2-deoxygluconolactam,
2-Acetamido-2-deoxygalactonolactam,
D-Glucaro-1,4:6,3-dilactam,
L-Idaro-1,4-lactam,
2,3,5-Tri-O-acetyl-D-glucaro-1,4-lactam,
2,5-Di-O-acetyl-D-Glucaro-1,4:6,3-dilactam,
D-glucaro-1,5-lactam ethyl ester;

xii. Chemical activators of protein kinase C enzymes, as described by Unilever in EP 0 334 585 chosen from diacylglycerols,
preferred examples of which include:
1,2-Dibutanoyl-rac-glycerol
1,2-Dihexanoyl-sn-glycerol
1,2-Dioctanoyl-rac-glycerol
1,2-Dioctanoyl-sn-glycerol
1,2-Didecanoyl-rac-glycerol
1-Oleoyl-2-acetyl-rac-glycerol
1-Oleoyl-2-acetyl-sn-glycerol
1-Stearoyl-2-arachidonoyl-sn-glycerol
1,2-Distearoyl-rac-glycerol
1,2-Dipentadecanoyl-sn-glycerol
1,2-dipentadecanoyl-rac-glycerol
1,2-Dipalmitoyl-rac-glycerol
1,2-Dipalmitoyl-sn-glycerol
1,2-Diseptadecanoyl-rac-glycerol
1,2-Dioleoyl-sn-glycerol
1,2-Dioleoyl-rac-glycerol
1,2-Diarachidonoyl-sn-glycerol
1,2-Dieicosanoyl-sn-glycerol
1,2-Didoeicosanoyl-rac-glycerol, and
1,2-Dioctaeicosanoyl-sn-glycerol.

xiii.Glycosaminoglycanase inhibitors, as described by Unilever in EP 0 348 184, chosen from aldonomonolactone or alduronomonolactone derivatives,
preferred examples of which aldonomonolactone derivatives include:
6-acetyl-galactono-1,4-lactone
6-propionyl-galactono-1,4-lactone
6-butyryl-galactono-1,4-lactone
2-propionamido-2-deoxygluconolactone
2-butyramido-2-deoxygluconolactone
2-propionamido-2-deoxygalactonolactone

2-butyramido-2-deoxygalactonolactone
6-propionyl-2-acetamido-2-deoxygluconolactone
diacetyl-6-propionyl-2-acetamido-2-deoxygluconolactone
6-butyryl-2-acetamido-2-deoxygalactonolactone
diacetyl-6-butyryl-2-acetamido-2-deoxygalactonolactone
2,3,5,6-tetraacetyl-galactono-1,4-lactone
2,3,5-triacetyl-6-propionylgalactono-1,4-lactone
triacetyl-2-propionamido-2-deoxygalactonolactone
triacetyl-2-butyramido-2-deoxygluconolactone
6-methyl-glucaro-1,4-lactone
2,3,5,6-tetramethyl-glucaro-1,4-lactone
6-methyl-2,3,5-triacetylglucaro-1,4-lactone
6-methyl-3-methyl-glucaro-1,4-lactone, and
6-methyl-3-acetyl-glucaro-1,4-lactone;
and a preferred example of which alduronomonolactone derivative is:
1,2,5-triacetyl-glucurono-6,3-lactone.
xiv. Glycosaminoglycanase inhibitors, as described by Unilever in EP 0 348 184, chosen from acylated monosaccharides,
preferred examples of which acylated monosaccharides include:
2-propionamido-2-deoxyglucose
1,3,4,6-tetraacetyl-2-propionamido-2-deoxyglucose
2-butyramido-2-deoxygalactose
1,3,4,6-tetraacetyl-2-butyramido-2-deoxygalactose
2-sulphamido-2-deoxygalactose
2-sulphamido-2-deoxyglucose
2-butyramido-2-deoxymannose
1,3,4,6-tetraacetyl-2-butyramido-2-deoxymannose
2-butyramido-2-deoxyglucose, and
1,3,4,6-tetraacetyl-2-butyramido-2-deoxyglucose.
xv. Esters of pyroglutamic acid, as described by Lever Brothers Company in US patent No. 4 774 255, preferred examples of which include:
pyroglutamic acid methyl ester
pyroglutamic acid ethyl ester
pyroglutamic acid n-propyl ester
pyroglutamic acid n-butyl ester
pyroglutamic acid n-hexyl ester
pyroglutamic acid n-heptyl ester
pyroglutamic acid n-octyl ester
pyroglutamic acid n-nonyl ester
pyroglutamic acid n-decyl ester
pyroglutamic acid n-undecyl ester
pyroglutamic acid n-dodecyl ester
pyroglutamic acid n-tridecyl ester
pyroglutamic acid n-tetradcyl ester
pyroglutamic acid n-hexadecyl ester
pyroglutamic acid n-octadecyl ester
pyroglutamic acid n-eicosyl ester
pyroglutamic acid iso-propyl ester
pyroglutamic acid 2-methylhexyl ester
pyroglutamic acid 2-ethylhexyl ester
pyroglutamic acid 3,7-dimethyloctyl ester
pyroglutamic acid 2-hexyldecyl ester
pyroglutamic acid 2-octyldodecyl ester
pyroglutamic acid 2,4,4-trimetyl-1-pentane ester
pyroglutamic acid methyloctyl ester
2-[pyroglutamoyloxy]-propionic acid
methyl-2-[pyroglutamoyloxy]-acetate
ethyl-2-[pyroglutamoyloxy]-n-propionate

ethyl-2-[pyroglutamoyloxy]-n-butyrate
ethyl-2-[pyroglutamoyloxy]-iso-butyrate
ethyl-2-[pyroglutamoyloxy]-n-valerate
ethyl-2-[pyroglutamoyloxy]-n-caproate
ethyl-2-[pyroglutamoyloxy]-n-heptylate
ethyl-2-[pyroglutamoyloxy]-n-caprylate
ethyl-2-[pyroglutamoyloxy]-n-pelargonate
ethyl-2-[pyroglutamoyloxy]-3-hydroxybutyrate
iso-propyl-2-[pyroglutamoyloxy]-n-propionate
iso-propyl-2-[pyroglutamoyloxy]-n-caprylate
n-propyl-2-[pyroglutamoyloxy]-n-propionate
n-propyl-2-[pyroglutamoyloxy]-n-caprylate
stearyl-2-[pyroglutamoyloxy]-n-propionate
12-hydroxystearyl-2-[pyroglutamoyloxy]-n-propionate
stearyl-2-[pyroglutamoyloxy]-n-stearate
palmityl-2-[pyroglutamoyloxy]-n-propionate
linoleyl-2-[pyroglutamoyloxy]-n-propionate
linoleyl-2-[pyroglutamoyloxy]-n-caprylate
lauryl-2-[pyroglutamoyloxy]-n-caprylate
stearyl-2-[pyroglutamoyloxy]-n-caprylate
glyceryl mono(2-[pyroglutamoyloxy]-n-propionate)
glyceryl mono(2-[pyroglutamoyloxy]-n-caprylate), and
glyceryl di(2-[pyroglutamoyloxy]-n-propionate).

xvi. hexosaccharic acids or an acylated hexosaccharic acids, or salts or esters thereof, as described by Unilever in EP 378 388

preferred examples of which include:
allosaccharic acid
altrosaccharic acid
glucosaccharic acid
mannosaccharic acid
gulosaccharic acid
idosaccharic acid
galactosaccharic acid
talosaccharic acid, and
their disodium salts.

xvii.aryl-substituted ethylenes as described by Unilever in EP 403 238,
preferred examples of which include:
1-carboxy-2-(4-hydroxyphenyl)ethylene
1,1-dicarboxy-2-(4-hydroxyphenyl)ethylene
1,1-dicyano-2-(4-hydroxyphenyl)ethylene
1-carboxy-2-(3,4-dihydroxyphenyl)ethylene
1,1-dicyano-2-(3-hydroxyphenyl)ethylene
1-cyano-1-carboxy-2-(2,5-dihydroxyphenyl)ethylene
1-carboxy-1-cyano-2-(3,4-dihydroxphenyl)ethylene
1,1-dicyano-2-(3,4-dihydroxyphenyl)ethylene
1,1-dicyano-2-(3-methoxy-4,5-dihydroxyphenyl)ethylene
1,1-dicyano-2-(3,4,5-trihydroxyphenyl)ethylene
1-amido-1-cyano-2-(3,4-dihydroxyphenyl)ethylene
1-thioamido-1-cyano-2-(3,4-dihydroxyphenyl)ethylene
1-cyano-2-(4-hydroxyphenyl)ethylene
1,1-dicyano-2-(3-hydroxy-4-nitrophenyl)ethylene
1,1-dicyano-2-hydroxy-2-(4-hydroxyphenyl)ethylene
1,1-dicyano-2-(3-methoxy-4-hydroxyphenyl)ethylene
1,1-dicyano-2-(3,5-dihydroxyphenyl)ethylene
1,1-dicyano-2-hydroxy-2-(3,4,5-trihydroxyphenyl)ethylene
1-carboxy-1-cyano-2-(4-methoxyphenyl)ethylene
1-carboxy-1-cyano-2-(4-fluorophenyl)ethylene
1-carboxy-1-cyano-2-(3-methoxy-4-hydroxyphenyl)ethylene

1-carboxy-1-cyano-2-(3,5-dimethoxy-4-hydroxyphenyl)ethylene
1-carboxy-1-cyano-2-(4-hydroxyphenyl)ethylene
1-carboxy-1-cyano-2-(4-phenylcarboxyaldehyde)ethylene, and
1-cyano-1-carboxy-2-(2,5-dihydroxyphenyl)ethylene

xviii. N-acylated amino acids as described by Unilever in EP 415 598.
Preferred examples of which include:

N-acetyl glycine
N-acetyl hydroxyproline
N-acetyl alanine
N-acetyl valine
N-acetyl leucine
N-acetyl isoleucine
N-acetyl phenylalanine
N-acetyl tyrosine
N-acetyl proline
N-acetyl serine
N-acetyl threonine
N-acetyl cysteine
N-acetyl cystine
N-acetyl methionine
N-acetyl tryptophan
N-lauroyl glycine
N-palmitoyl glycine
N-myristoyl glycine
N-lauroyl hydroxyproline
N-octanoyl glycine
N-octanoyl hydroxyproline
N-hexanoyl glycine
N-acetyl aspartic acid
N-lauroyl aspartic acid
N-palmitoyl aspartic acid
N-octanoyl aspartic acid
N-acetyl glutamic acid
N-lauroyl glutamic acid
N-palmitoyl glutamic acid
N-octanoyl glutamic acid
N-acetyl arginine
N-acetyl lysine
N-acetyl histidine
N-acetyl ornithine
N-acetyl hydroxylysine
N-acetyl citrulline
N-lauroyl lysine
N-lauroyl citrulline
N-myristoyl citrulline
N-myristoyl ornithine
N-octanoyl lysine, and
N-octanoyl citrulline.

(b) Penetration Enhancers

As has been stated earlier, the presence of a penetration enhancer can potentiate the benefit of the substituted DOPA by improving its delivery through the stratum corneum to its site of action, particularly in the immediate environment of the hair follicle close to the dermal papilla.

The penetration enhancer can accordingly function in a variety of ways. It can for example, improve the distribution of the substututed DOPA on the skin surface or, it can increase its partition into the skin from the composition when applied topically, so aiding its passage to its site of action. Other mechanisms enhancing the benefit of the hair growth promoter may also be involved.

Examples of penetration enhancers include:
2-methyl propan-2-ol
Propan-2-ol
Ethyl-2-hydroxypropanoate
Hexan-2,5-diol
POE(2) ethyl ether
Di(2-hydroxypropyl) ether
Pentan-2,4-diol
Acetone
POE(2) methyl ether
2-hydroxypropionic acid
2-hydroxyoctanoic acid
Propan-1-ol
1,4 Dioxane
Tetrahydrofuran
Butan-1,4-diol
Propylene glycol dipelargonate
Polyoxypropylene 15 stearyl ether
Octyl alcohol
POE ester of oleyl alcohol
Oleyl alcohol
Lauryl alcohol
Dioctyl adipate
Dicapryl adipate
Diisopropyl adipate
Diisopropyl sebacate
Dibutyl sebacate
Diethyl sebacate
Dimethyl sebacate
Dioctyl sebacate
Dibutyl suberate
Dioctyl azelate
Debenzyl sebacate
Dibutyl phthalate
Dibutyl azelate
Ethyl myristate
Dimethyl azelate
Butyl myristate
Dibutyl succinate
Didecyl phthalate
Decyl oleate
Ethyl caproate
Ethyl salicylate
Isopropyl palmitate
Ethyl laurate
2-ethyl-hexyl pelargonate
Isopropyl isostearate
Butyl laurate
Benzyl benzoate
Butyl benzoate
Hexyl laurate
Ethyl caprate
Ethyl caprylate
Butyl stearate
Benzyl salicylate
2-hydroxypropanoic acid
2-hyroxyoctanoic acid,
Dimethyl sulphoxide

N,N-Dimethyl acetamide
N,N-Dimethyl formamide
2-Pyrrolidone
1-Methyl-2-pyrrolidone
5-Methyl-2-pyrrolidone
1,5-Dimethyl-2-pyrrolidone
1-Ethyl-2-pyrrolidone
Phosphine oxides
Sugar esters
Tetrahydrofurfural alcohol
Urea
Diethyl-m-toluamide, and
1-Dodecylazacyloheptan-2-one

The amount of activity enhancer, when employed in accordance with the invention, will normally be from 0.1 to 50%, preferably from 0.5 to 25% and most preferably from 0.5 to 10% by weight of the composition.

Other Cosmetic Adjuncts

Examples of conventional adjuncts which can optionally be employed include preservatives, such as para-hydroxy benzoate esters; sunscreens, such as butyl methoxy dibenzoyl methane (PARSOL 1789) and octyl methoxy cinnamate (PARSOL MCX); antioxidants, such butyl hydroxy toluene; humectants, such as glycerol, sorbitol, 2-pyrrolidone-5-carboxylate, dibutylphthalate, gelatin, polyethylene glycol, such as PEG 200-600; buffers, such as lactic acid together with a base such as triethanolamine or sodium hydroxide; waxes, such as beeswax, ozokerite wax, paraffin wax; plant extracts, such as Aloe vera, cornflower, witch hazel, elderflower, cucumber; thickeners; activity enhancers; colourants; and perfumes. Cosmetic adjuncts can form the balance of the composition.

Use of the Composition

The composition according to the invention is intended primarily for topical application to human skin particularly the scalp, to stimulate darkening of hair on regrowth. In use, a small quantity of the composition, for example from 1 to 5ml, is applied to exposed areas of the skin, particularly the scalp, from a suitable container or applicator and, if necessary, it is then spread over and/or rubbed into the skin using the hand or fingers or a suitable device. When the composition is a shampoo and/or a hair conditioner, it can be applied to the hair (and scalp) in the conventional manner and either rinsed with water or left in place without rinsing. Repeated applications will generally achieve best results.

The composition is also suitable for topical application to the skin, in order to promote darkening of the skin to mimic tanning which normally follows exposure to sunlight or artificial ultra-violet radiation. In use a small quantity, for example 1 to 2ml, is applied to the skin and rubbed in. Skin darkening will generally occur gradually after repeated application, for example twice daily over a period of seven to ten days.

Demonstration of Darkening of Hair on Regrowth

In order to demonstrate that the substituted DOPAs are capable of darkening hair on regrowth, an in vitro test was established in which isolated living human hair follicles were contacted with selected substituted DOPAs for a period of up to seven days, any darkening being recorded following daily observations by trained assessors.

The isolation of individual hair follicles and their culture will now be described.

Isolation of the hair follicle from skin

The method of testing includes the important step of isolating hair follicles having an undamaged hair bulb from skin, preferably from human skin, by microdissection, but preferably without the enzymic pretreatment advocated by other workers in this field.

Separation of hair follicles with an intact, undamaged and fully functioning hair bulb, even without enzymic pretreatment, is virtually impossible by normal microdissection methods, in view of the degree of adhesion that exists between the hair follicle and the skin tissue where it passes through the dermis. Thus, separation of dermis from the hair follicle almost invariably causes damage to the hair bulb.

The critical step of separating the hair follicle with intact undamaged hair bulb from the subcutaneous fatty tissue in which it is situated accordingly involves severing the hair shaft of the follicle at a point below the epidermis or skin surface, so as to leave the hair bulb intact and undamaged while still bearing a portion of the hair shaft.

Preferably, the hair shaft of the follicle is severed at the dermal-subcutaneous fat interface.

Any suitable cutting instrument can be employed to sever the hair shaft in this manner, but a keratotome or a scalpel are preferred.

The hair bulb with a hair shaft stump attached is then isolated from the skin by mechanically separating the hair from loosely adhering subcutaneous fat which normally surrounds the hair bulb. This is achieved after the dermis or upper layer of the skin has been separated and removed, to avoid damaging the hair bulb as it is pulled away.

The hair bulb together with the hair shaft stump attached, is then transferred in an otherwise undamaged and fully functioning, viable state to a nutrient medium.

Culture of the isolated hair follicle

The hair follicles isolated by the technique described above are transferred to a suitable culture medium for subsequent testing of substances that can then influence their future development.

In accordance with the preferred method of culture, isolated hair follicles were maintained in 500μl of Williams E medium, containing the test substance (substituted DOPA) at a concentration of 1mM, at 37°C in an atmosphere of 5% $CO_2$ + 95% air in individual wells of a 24 multiwell dish (Corning), which permitted detailed observations of the colour and measurements to be made of individual hair follicles.

Williams E medium is available from FLOW Laboratory under Catalogue No. 12-502. The formula of Williams E medium is described by Williams GM, et al., in Experimental Cell Research 69 (1971) at page 106.

Hair shaft length measurements and colour changes, i.e. darkening, were made on these follicles, using a Nikon Diaphot inverted binocular photo microscope with eye piece measuring graticules, over seven days in culture.

Furthermore, photographic evidence showed that this darkening, if any, and increase in length is not associated with any disruption of hair follicle architecture.

It is also possible to employ several different biochemical and morphometric analyses to study the effect of a substance on the viable hair follicle. For example, including tritiated thymidine in the growth medium, then subsequently isolating the follicles from the growth medium and examining them by autoradiography shows that in freshly isolated hair follicles, the typical pattern of cell division is taking place, with the majority of thymidine uptake occurring in the matrix cells of the hair follicle bulb, adjacent to the dermal papilla. Over seven days' maintenance, this pattern remains constant.

Experimental testing of substituted DOPAs for this ability to darken hair on regrowth

The techniques described herein were employed to test several substances for their ability to darken hair as hair growth proceeds.

Groups of eight freshly isolated hair follicles were maintained in a Williams E medium under the conditions stated above. For a control group of follicles the medium contained no test substance. For other groups of follicles the medium contained the test substance at 0.1mM concentration of 1mM concentration, In all cases the growth medium additionally contained L-glutamine (2mM), Penicillin (50 IU/ml), streptomycin (50 ug/ml), insulin (10 ug/ml) and hydrocortisone (10 ng/ml).

The follicles were photographed at the start of the incubation period and again after incubation for two or four days. The growth and morphology of the follicles was monitored during the culture period.

With some of the test substances the follicles were observed to darken and the hair stump was observed to grow from the follicles as a hair shaft with dark colour. In the case of a control a hair shaft with little or no colour grew and the follicle did not darken. Moreover, some other substances used as a comparison also failed to cause darkening of the hair shaft or the follicles.

The materials tested and the results observed were as follows:

| AGENT | DARKENING OBSERVED |
|---|---|
| 0.1mM Dihydroxyphenylserine | YES (after 48 hrs) |
| 0.1mM 2-methyl-3-(3,4-dihydroxyphenyl)alanine | YES (after 48 hrs) |
| 1mM 3-methoxy-4-hydroxyphenyl alanine | YES (96 hrs) |
| 1mM methyl-3,4-diacetyl-L-DOPA hydrochloride | YES (96 hrs) |
| 1mM 3,4-diacetyl-L-DOPA hydrochloride | YES (96 hrs) |
| 1mM Ethyl D-L-3,4-DOPA hydrochloride | NO |
| 1mM Butyl D-L-3,4-DOPA hydrochloride | NO |
| 1mM Hexyl D-L-3,4-DOPA hydrochloride | NO |
| 1mM Octyl D-L-3,4-DOPA hydrochloride | NO |

Of the various materials tested, the first two, i.e. dihydroxyphenylserine and 2-methyl-3-(3,4-dihyroxyphenyl)alanine were preferred since they did not appear to inhibit or damage the follicles whereas the other materials tested did appear to cause some damage to the follicles.

Melanin formation by these follicles was assessed. The procedure was based on that of Gardon and Gilchrest, 1989. For each assay 12 cultured follicles were placed in 500ul of 1M NaOH and incubated overnight at 4°C. The follicles were then homogenised and centrifuged at 14,000 rpm for 2 min. The supernatant was removed, 200ul samples added to a 96 well plate and the melanin content then determined spectrophotometrically at 540nm. A standard curve was also prepared from synthetic melanin dissolved in 1M NaOH. The protein content of each sample was also determined by the Folin & Lowry method, with the absorbance being read at 540nm. Samples generally needed a 1:100 dilution to fit within the range of the standard curve, prepared with bovine serum albumin. Results were expressed as ug melanin/mg soluble protein.

The results were as follows:

| Test substance | μg Melanin/mg soluble protein |
|---|---|
| Dihydroxyphenylserine | 6.31 |
| 2-methyl-3-(3,4-dihydroxyphenyl)alanine | 8.36 |
| None (control) | 1.11 |

## PRODUCT FORM AND PACKAGING

The topical composition of the invention can be formulated as a shampoo or hair conditioner, or as a lotion having a viscosity of from 4,000 to 10,000 mPas, a fluid cream having a viscosity of from 20,000 to 100,000 mPas, or above. The composition can be packaged in a suitable container to suit its viscosity and intended use by the consumer.

For example, a shampoo, conditioner, lotion or fluid cream can be packaged in a bottle or a roll-ball applicator or a propellant-driven aerosol device or a container fitted with a pump suitable for finger operation. When the composition is a cream, it can simply be stored in a non-deformable bottle or squeeze container, such as a tube or a lidded jar.

The invention accordingly also provides a closed container containing a cosmetically acceptable composition as herein defined.

## EXAMPLES

The invention is further illustrated by the following examples.

Example 1

This example illustrates a lotion according to the invention.

| Ingredient | % w/w |
|---|---|
| 3,4-dihydroxyphenylserine | 2 |
| silicone surfactant | 10 |
| volatile siloxane | 14 |
| mineral oil | 1.5 |
| 2-hydroxy octanoic acid | 1 |
| 2-hydroxy propanoic acid | 5 |
| butylene glycol | 10 |
| sodium chloride | 2 |
| 1-proline | 0.1 |
| neutralising agent | qs |
| preservative | qs |
| perfume | qs |
| water | qs |

Example 2

This example illustrates a fluid cream according to the invention.

| Ingredient | % w/w |
|---|---|
| 2,4,5-trihydroxyphenylalanine | 3 |
| volatile siloxane (DC 345) | 8.2 |
| silicone surfactant (DC 3225C) | 12 |
| petroleum jelly | 0.5 |
| mineral oil | 1.5 |
| Parsol MCX (octyl methoxycinnamate) | 3 |
| sodium chloride | 2 |
| butylene glycol | 10 |
| 1-proline | 0.1 |
| 2-hydroxy octanoic acid | 1 |
| 2-hydroxy propanoic acid | 5 |
| neutralising agent | qs |
| preservative | qs |
| perfume | qs |
| water | qs |

Example 3

This example illustrates a cream according to the invention.

| Ingredient | % w/w |
|---|---|
| 2-methyl-3-(3,4-dihydroxyphenylalanine) | 2 |
| volatile siloxane (DC 345 Fluid) | 8.2 |
| silicone surfactant (DC 3225C) | 12 |
| mineral oil | 1.5 |
| petroleum jelly | 0.5 |
| Parsol MCX (octyl methoxycinnamate) | 1.5 |
| 2-hydroxyoctanoic acid | 1 |
| 2-hydroxypropanoic acid | 5 |
| sodium chloride | 2 |
| butylene glycol | 10 |
| 1-proline | 0.1 |
| neutralising agent (aqueous phase to 4.5) | q.s. |
| preservative | q.s. |
| perfume | q.s. |
| water | to 100 |

Example 4

This example illustrates a lotion according to the invention.

| Ingredient | % w/w |
|---|---|
| methoxytyrosine | 1 |
| silicone surfactant (DC 3225C) | 10 |
| volatile siloxane (DC 345) | 14 |
| mineral oil | 1.5 |
| Parsol MCX | 3 |
| butylene glycol | 10 |
| sodium chloride | 2 |
| 1-proline | 0.1 |
| 2-hydroxy octanoic acid | 1 |
| 2-hydroxy propanoic acid | 5 |
| neutralising agent | qs |
| perfume | qs |
| preservative | qs |
| water | qs |

Other analogues of DOPA in accordance with this invention may be substituted in the Examples. 2-methyl-3-(3,4-dihydroxy)phenyl alanine or dihydroxyphenylserine may be used as alternatives to the methoxytyrosine in the above lotion.

Example 5

This example illustrates a sunscreen cream in accordance with the invention.

| Ingredient | % w/w |
|---|---|
| 2-methyl-3-(3,4-dihydroxy)phenylalanine | 2 |
| Polyoxyethylene (2) stearyl alcohol | 3 |
| Polyoxyethylene (21) stearyl alcohol | 2 |
| cetyl alcohol | 1.5 |
| soft white paraffin | 1.5 |
| silicone fluid 200 | 5 |
| liquid paraffin | 8 |
| glycerin | 2 |
| preservatives | 0.5 |
| water | to 100 |

Example 6

This example also illustrates a cream in accordance with the invention.

| Ingredients | | % w/w |
|---|---|---|
| 3,4-dihydroxyphenylserine | | 2 |
| cetyl dimethicone copolyol | ) | |
| cetyl dimethicone | ) * | 5 |
| polyglyceryl-3-oleate | ) | |
| hexyl laurate | ) | |
| isopropyl myristate | | 13.5 |
| beeswax | | 3 |
| silicone fluid 200 | | 5 |
| preservatives | | 0.5 |
| water | | to 100 |

*Available as ABIL W508 ex Goldschmidt

Example 7

This example illustrates a lotion according to the invention.

| Ingredient | % w/w |
|---|---|
| 2,4,5-trihydroxyphenylalanine | 2 |
| silicone surfactant | 10 |
| volatile siloxane | 14 |
| mineral oil | 1.5 |
| 2-hydroxy octanoic acid | 1 |
| 2-hydroxy propanoic acid | 5 |
| butylene glycol | 10 |
| sodium chloride | 2 |
| amino acid | 0.1 |
| neutralising agent | qs |
| preservative | qs |
| perfume | qs |
| water | qs |

Example 8

This example illustrates a lotion according to the invention.

| Ingredient | % w/w |
|---|---|
| 2-methyl-3-(3,4-dihydroxyphenylalanine) | 3 |
| silicone surfactant | 10 |
| volatile siloxane | 14 |
| mineral oil | 1.5 |
| 2-hydroxy octanoic acid | 1 |
| 2-hydroxy propanoic acid | 5 |
| butylene glycol | 10 |
| sodium chloride | 2 |
| amino acid | 0.1 |
| neutralising agent | qs |
| preservative | qs |
| perfume | qs |
| water | qs |

Example 9

This example illustrates a lotion according to the invention.

| Ingredient | % w/w |
|---|---|
| methoxytyrosine | 1 |
| silicone surfactant | 10 |
| volatile siloxane | 14 |
| mineral oil | 1.5 |
| 2-hydroxy octanoic acid | 1 |
| 2-hydroxy propanoic acid | 5 |
| butylene glycol | 10 |
| sodium chloride | 2 |
| amino acid | 0.1 |
| neutralising agent | qs |
| preservative | qs |
| perfume | qs |
| water | qs |

Example 10

This example illustrates an anhydrous formulation in accordance with the invention.

| Ingredient | % w/w |
|---|---|
| dihydroxyphenylserine | 5 |
| Iso-propyl alcohol | 10 |
| ethyl hexyl palmitate | 8.7 |
| antioxidant | 0.1 |
| volatile silicone | to 100 |

Example 11

This example illustrates a shampoo according to the invention:

| | % wt |
|---|---|
| Ammonium lauryl sulphate/ Ammonium lauryl ether sulphate | 8.00 |
| Cocodiethanolamide | 3.00 |
| Butyl hydroxytoluene (BHT) | 0.05 |
| BRIPHOS 03D [1] | 1.05 |
| 3,4-dihydroxyphenylserine | 5.00 |
| Hydrolysed silk protein | 0.10 |
| CARBOPOL 1342 | 0.40 |
| Polyethoxylated lanolin | 0.30 |
| JAGUAR C13S | 0.30 |
| TIMIRON MP-1005 | 0.06 |
| Triethanolamine | 0.80 |
| Silicone emulsion (50%) [2] | 6.00 |
| Perfume, preservative | qs |
| Water | to 100 |

1. BRIPHOS 03D is a mixture of esters of phosphoric acid and the polyethylene glycol ether of oleyl alcohol.

2. Silicone emulsion comprises 50% by weight silicone oil (60,000 cs), 4% by weight cetostearyl alcohol and 25% by weight SLES 2EO.

The ammonium lauryl sulphate/ammonium lauryl ether sulphate and the BHT were heated in the main vessel to 5°C to melt the BHT, with constant stirring.

CARBOPOL 1342 was dispersed with stirring in 50% of the water, and the resulting dispersion added to the main vessel.

The polyethoxylated lanolin was melted at 70 to 75°C and added to the main vessel.

The cocodiethanolamide and BRIPHOS 03D were added to the main vessel with stirring.

JAGUAR C13S was dispersed in 35% of the water and added to the main vessel with high shear mixing.

Preservative, hydrolysed silk protein, triethanolamine and perfume were added to the main vessel, followed by the silicone emulsion, and finally the TIMIRON MP-1005.

The shampoo composition of Example 11 was found to be stable at 0°C and at ambient temperature after 6 months storage. Some separation was seen after storage at 37°C and 45°C for 4 months.

Example 12

This example also illustrates a shampoo according to the invention.

| | % wt |
|---|---|
| Ammonium lauryl sulphate/Ammonium lauryl ether sulphate | 8.00 |
| Cocodiethanolamide | 3.00 |
| BHT | 0.05 |
| BRIPHOS 03D | 1.05 |
| 2,4,5-trihydroxyphenylalanine | 4.00 |
| Hydrolysed silk protein | 0.10 |
| Xanthan gum | 0.40 |
| Polyethyoxylated lanolin | 0.30 |
| JAGUAR C13S | 0.30 |
| TIMIRON MP-1005 | 0.06 |
| Triethanolamine | 0.80 |
| Silicone emulsion (50%) | 6.00 |
| Perfume, preservative | qs |
| Water | to 100 |

The ALS/ALES mixture was placed in the main vessel. JAGUAR C13S was dispersed in 90% of the water, and added to the main vessel with high shear mixing.

The polyethoxylated lanolin was melted and added to the main vessel.

Cocodiethanolamide and BRIPHOS 03D were mixed and added slowly to the main vessel. The xanthan gum was added with mixing. The hydrolysed silk protein and triethanolamine were added. The preservative was added. The BHT was dissolved in the perfume and added to the main vessel.

The silicone emulsion was added, followed by the TIMIRON MP-1005, with rapid stirring.

The shampoo composition of Example 12 was found to be stable at 0°C, at ambient temperature and at 37°C after storage for 6 months.

Example 13

This example also illustrates a shampoo according to the invention.

| | % wt |
|---|---|
| Ammonium lauryl sulphate/ Ammonium lauryl ether sulphate | 8.00 |
| Polyethoxylated lanolin | 0.30 |
| CARBOPOL 1342 | 0.40 |
| Cocodiethanolamide | 3.00 |
| BRIPHOS 03D | 1.05 |
| 2-methyl-3-(3,4-dihydroxyphenylalanine) | 6.00 |
| BHT | 0.05 |
| JAGUAR C13S | 0.30 |
| Hydrolysed silk protein | 0.10 |
| Triethanolamine | 0.80 |
| TIMIRON MP-1005 | 0.06 |
| TORAY BY-22022 [3] | 6.00 |
| Perfume, preservative | qs |
| Water | to 100 |

3. TORAY BY-22022 is a commercially available silicone emulsion (50% by weight silicone oil).

The ALS/ALES mixture and BHT were added to the main vessel and heated to melt the BHT. The hydrolysed silk protein was added. CARBOPOL 1342 was dispersed in 50% of the water and added to the main vessel with stirring. Cocodiethanolamide was added very slowly, followed by BRIPHOS 03D.

JAGUAR C13S was dispersed in 45% of the water and added to the main vessel with high shear mixing, Triethanolamine and hydrolysed silk protein were added followed by perfume and preservative. The silicone emulsion was then added, and finally TIMIRON MP-1005 was added with stirring.

The shampoo composition of Example 13 was found to be stable at 0°C, after storage for 6 months.

## Claims

1. A preserved composition suitable for topical application to mammalian skin for darkening hair on regrowth which comprises:
   i. an effective amount of a substituted DOPA as a melanin precursor, chosen from:
      (a) mono and/or di-substituted ester and formate derivatives of DOPA having the structure (10):

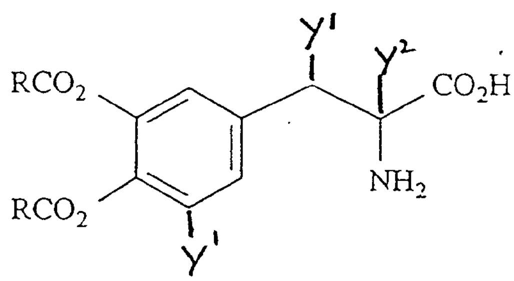

(10)

where R is -H, a branched or unbranched, alkyl or alkenyl group having from 1 to 20 carbon atoms, or an amino acid or peptide fragment,
$Y^1$ is -H or -OH, and
$Y^2$ is -H or $-CH_3$;
(b) mono and/or di-substituted carbonate derivatives of DOPA having the structure (11):

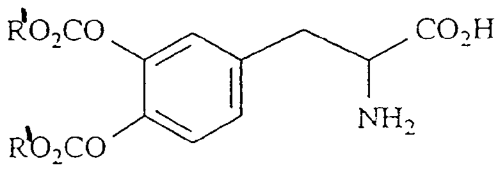

(11)

where $R^1$ is a branched or unbranched, alkyl or alkenyl group having from 1 to 20 carbon atoms;
(c) mono and/or di-substituted urethane derivatives of DOPA having the structure (12):

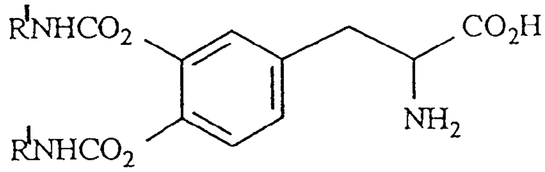

(12)

(d) mono and/or di-substituted ether derivatives of DOPA having the structure (13):

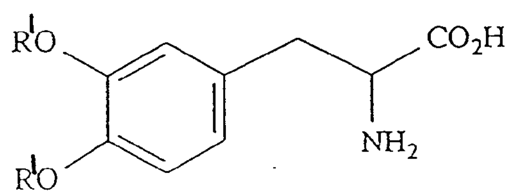

(13)

(e) mono and/or di-substituted phosphate and/or sulphate derivatives of DOPA having the structure (14):

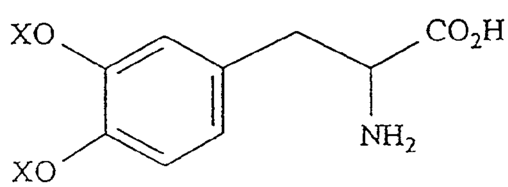

(14)

where one X is $-PO_3H_2$ or $-SO_3H$ and the other X is -H, $-PO_3H_2$ or $-SO_3H$;
(f) acetal and ketal derivatives of DOPA having the structure (15):

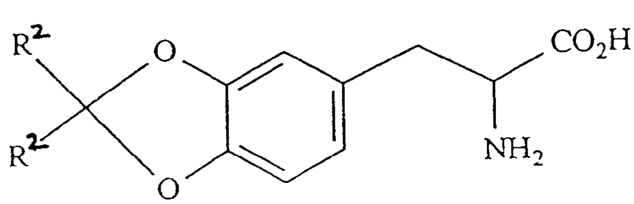

(15)

where $R^2$ is chosen from -H, alkyl and phenyl;
(g) cyclic carbonate derivatives of DOPA having the structure (16):

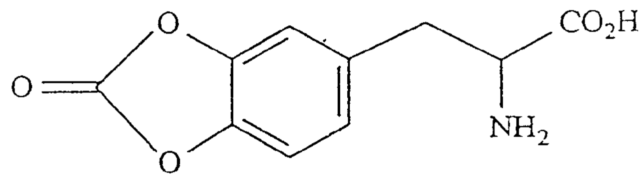

(16)

(h) amino substituted derivatives of DOPA having the structure (17):

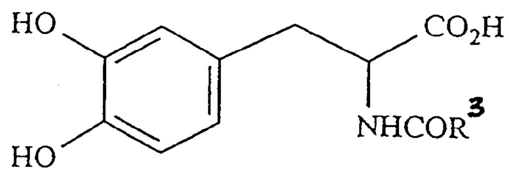

(17)

where $R^3 = R^1$ or an amino acid residue;
(i) carboxylate substituted derivatives of DOPA having the structure (18):

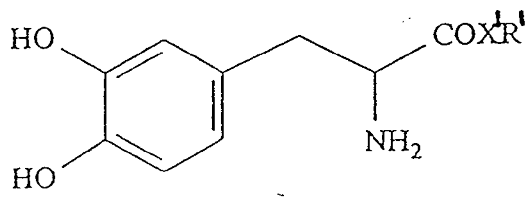

(18)

where $X^1$ is an NH amide (especially an amino acid or peptide) linkage;
(j) linked amino and carboxylate substituted derivatives of DOPA having the structure (19):

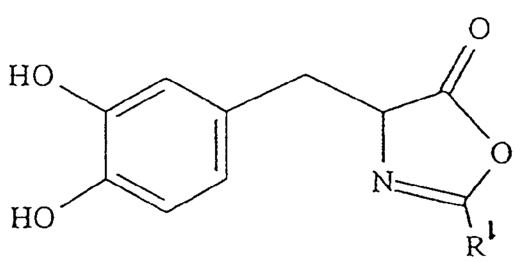

(19)

(k) structural analogues of DOPA having the structure (20):

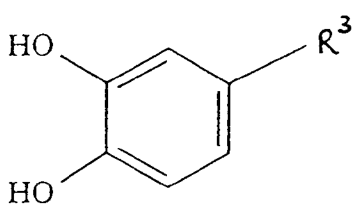

(20)

where $R^3$ is chosen from:

(i)

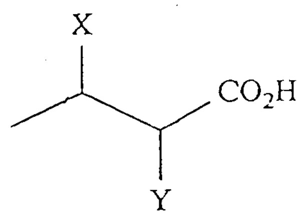

where $X^2$ is the same or different and is chosen from -H, -OH, $-NH_2$ or -SH

(ii)

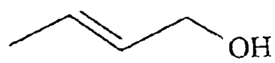

(iii)

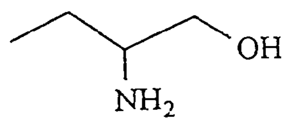

(iv)

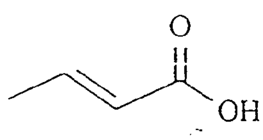

(l) structural analogues of DOPA having the structure (21):

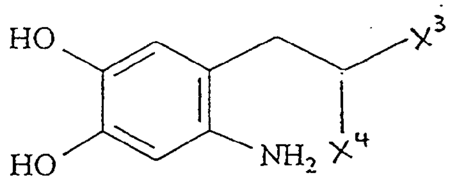

(21)

where

$X^3$ is -OH or $OR^1$

$X^4$ is =O or $OR^1$

(m) C-homologues of DOPA having the structure (22):

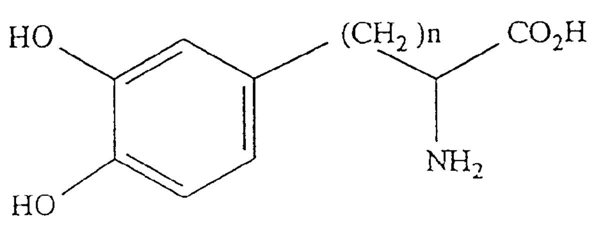

(22)

where n is an integer of from 1 to 3;

(n) short chain and hetero atom analogues of DOPA having the structure (23):

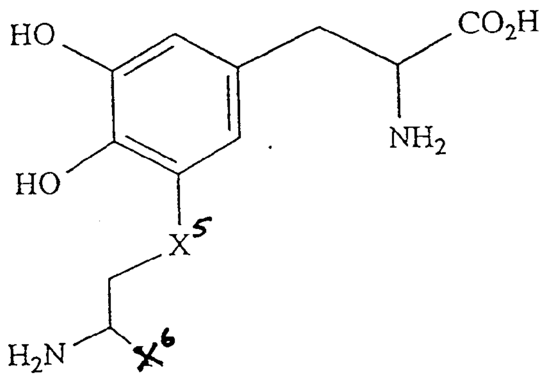

(23)

where
$X^5$ is S, O or NH
$X^6$ is H or COOH; and
(o) a glutathione derivative having the structure (24):

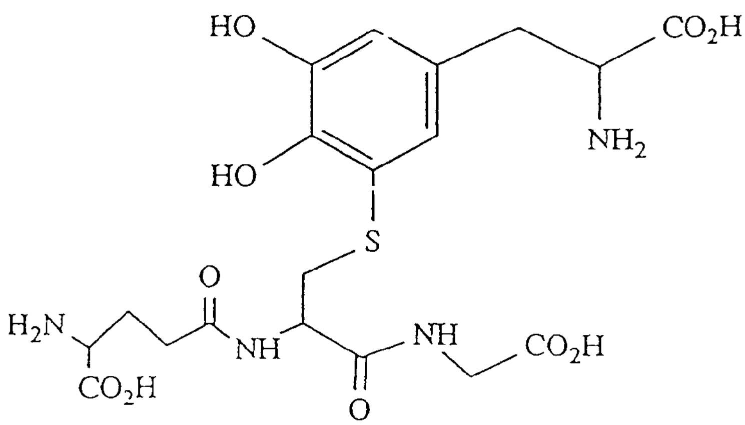

(24)

ii) a cosmetically acceptable vehicle for the substituted DOPA.

2. A composition according to claim 1, in which the substituted DOPA is chosen from:
3,4-dihydroxyphenylserine, having the structure (50):

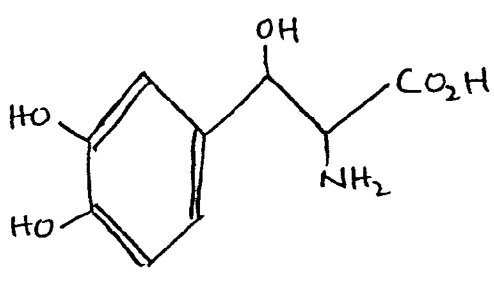

(50)

2,4,5,-trihydroxyphenylalanine, having the structure (51):

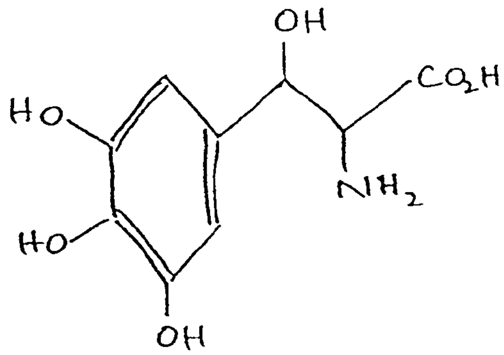

(51)

2-methyl-3-(3,4-dihydroxyphenylalanine), having the structure (52):

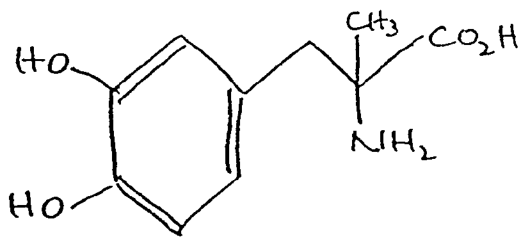

(52)

3. A composition according to claim 1 or 2, in which the substituted DOPA forms from 0.0001 to 99.9% by weight.

4. A composition according to claim 1, 2 or 3, in which the substituted DOPA forms from 1 to 50% by weight.

5. A composition according to any preceding claim, which further comprises a surfactant.

6. A composition according to any preceding claim, which further comprises a hair conditioning agent.

7. Use of a substituted DOPA as defined in claim 1, together with a major proportion of a cosmetically acceptable vehicle for the substituted DOPA, for darkening hair on regrowth following topical application to skin, particularly the scalp.

8. Use of a substituted DOPA as defined in claim 1, together with a major proportion of a cosmetically acceptable vehicle for the substituted DOPA, for darkening skin by repeated application thereto.

# Melanogenesis Pathway

Figure 1